# EUROPÄISCHE PATENTSCHRIFT

(11) **EP 1 097 234 B1**
(45) Veröffentlichungstag und Bekanntmachung des Hinweises auf die Patenterteilung: **06.04.2005**
(21) Anmeldenummer: 99947210.3
(22) Anmeldetag: 12.07.1999
(51) Int. Cl.: C12N 15/85, C07K 14/47, C12N 15/86, A61K 48/00

(54) **VERFAHREN ZUR VERBESSERUNG DES GENTRANSFERS VON GENETISCHEM MATERIAL IN SÄUGERZELLEN DURCH DIE VERWENDUNG VON P21 (WAF-1)**
METHOD FOR IMPROVING THE GENE TRANSFER OF GENETIC MATERIAL IN MAMMALIAN CELLS THROUGH THE USE OF P21 (WAF-1)
Procédé pour ameliorer le transfert de matériel genétique dans des cellules de mammifère grâce à l'utilisation de p21 (WAF-1)

(30) Priorität: 10.07.1998 DE 19830874
(43) Veröffentlichungstag der Anmeldung: 09.05.2001
(73) Patentinhaber: MAX-DELBRÜCK-CENTRUM FÜR MOLEKULARE MEDIZIN, 13125 Berlin (DE); Dörken, Bernd, Prof. Dr., 14055 Berlin (DE)
(72) Erfinder: DÖRKEN, Bernd, D-14055 Berlin (DE); WOLFF, Gerhard, D-10437 Berlin (DE); SCHUMACHER, Axel, D-13156 Berlin (DE)
(74) Vertreter: Baumbach, Friedrich, Dr.
(86) Internationale Anmeldenummer: PCT/DE1999/002180
(87) Internationale Veröffentlichungsnummer: WO 2000/003027

(56) Entgegenhaltungen:
- WO-A-97/11720

## Beschreibung

Die Erfindung betrifft ein Verfahren zur Stabilisierung und Verbesserung des Gentransfers in Säugerzellen durch Überexpression des Zellzyklusregulators p21, einem Hemmer von Zyklin-abhängigen Kinasen (CDK).

Unter Verwendung an sich bekannter Gentransfertechniken wird ein Gen und/oder dessen cDNA in eine beliebige Zielzelle eingebracht, um ein korrespondierendes Genprodukt zu exprimieren. Es existieren verschiedene Methoden, so z.B. Übertragung reiner DNA oder unter Verwendung viraler und nichtviraler Vektoren. Diese Systeme transferieren die Expressionskassette, welche das zu übertragende genetische Material und gegebenenfalls einen Promotor enthält, in die Zielzelle. Abhängig vom Endziel des Gentransfers, gibt es eine große Vielfalt an Verfahren zum Gentransfer und Expression eines beliebigen Transgens in eine Zielzelle. Für die Expression des Transgens wird die "Wirtszellmaschinerie" dazu genutzt, die genomische Information in das korrespondierende Protein zu überführen.

Eine Limitation der Effizienz solcher Gentransfertechnik ist jedoch die Induktion des programmierten Zelltods (Apoptose). Diese Zytopathogenität ist von verschiedenen Faktoren abhängig, wie z.B. Typ und Dosierung des verwendeten Vektors oder des Expressionskassetten-Konstruktes.

Immunologische Nebeneffekte begrenzen die Effizienz z.B. des Adenovirus-vermittelten Gentransfers in-vivo. Die Induktion der Apoptose jedoch ist ein Hauptproblem sowohl für die in-vivo als auch die in-vitro Effizienz.

Es ist bekannt, dass durch die Expression des Zellzyklusregulators p21 in humanen Zellen der Zellzyklus unterbrochen und der Zelltod induziert wird. So konnte z.B. gezeigt werden, dass die Expression von p21 in vivo zur Hemmung des Wachstums von Tumorzellen führt (WO 97 11720 A). Die Verwendung von p21 zur Apoptosehemmung wird hingegen nicht beschrieben.

Der Erfindung lag deshalb die Aufgabe zugrunde, ein Verfahren zu entwickeln, das die Induktion der Apoptose der Zielzelle bei und/oder nach einem Gentransfer verhindert und somit den Gentransfer in eine beliebige Säugerzelle verbessert und/oder verlängert.

Die Aufgabe wird dadurch gelöst, daß das genetische Material, z.B. ein therapeutisches Gen, das die DNA bzw. cDNA eines Gens darstellt, welches bei der zu behandelnden Krankheit defekt oder deletiert ist, nach an sich bekannten Methoden, ggf. unter Verwendung von Promotoren, in eine Zielzelle transferiert und zur Expression gebracht wird. Gleichzeitig wird erfindungsgemäß die cDNA des Zellzyklusregulationsproteins p21 zur Expression gebracht, indem sie gleichzeitig und/oder vorher transferiert worden war.

p21 ist ein bekannter Zellzyklusregulator, der als Hemmer von Zyklin-abhängigen Kinasen (CDK) den Wiedereintritt senescenter Zellen in den Zellzyklus verhindert. Diese Funktion des p21 schließt verschiedene Mechanismen ein, wie Hypophosphorylierung des Retinoblastom Genproduktes (Rb), Bindung an 'proliferating cell nuclear antigen' (PCNA), Bindung an CDK-Zyklin-Komplexen wie Zyklin D-CDK4, Zyklin E-CDK2 und Zyklin A-CDK2. Während die Wechselwirkung von p21 mit PCNA die DNA-Replikation blockiert, verhindert die Wechselwirkung mit CDK-Zyklin-Komplexen den Zellzyklusbeginn durch G1 Arrest. Die Gegenwart von p21 und seiner zellulären Funktion ist von Lebenswichtigkeit für das Überleben der Zelle. Das zeigt sich darin, daß kaum Mutationen des Proteins existieren, die lebensfähig sind.

Das erfindungsgemäße Verfahren verhindert überraschend die Apoptoseinduktion der Zielzelle nach dem Gentransfer durch die Überexpression von p21.

Bekanntermaßen replizieren Eukaryonten-Zellen ihr Genom nur während eines umschriebenen Zeitabschnittes, der als DNA - Synthesephase oder kurz als S-Phase bezeichnet wird. Die Phasen des Zellzyklus umfassen vier Phasen: G1-Phase, S-Phase, G2-Phase und Mitose. Ihre Zeiten sind relativ konstant: Die G1-Phase ist von unterschiedlicher Länge: in schnell proliferierenden Zellen liegt sie zwischen 2 und 20 Stunden, S-Phase: 6-10 Stunden; G2-Phase: 2-4 Stunden, Mitose: 3-4 Stunden.

Für den Gentransfer werden an sich übliche Übertragungsmethoden eingesetzt, wobei entweder die reine DNA übertragen wird oder sie in entsprechende Vektoren verpackt wird, die viral oder nichtviral sein können. Vorzugsweise werden virale Vektoren verwendet, wie die DNA von Adenoviren, Retroviren, Baculoviren, Paroviren und/oder Herpesviren u.a..

Gemäß der Erfindung besteht eine Ausführungsvariante darin, z.B. die DNA eines beliebigen therapeutischen Gens und die cDNA von p21 auf dem gleichen Vektor zu lokalisieren. Anschließend wird das Vektorsystem in die Zielzelle eingebracht, wobei das gewünschte Protein und p21 exprimiert werden.

Eine andere bevorzugte Ausführungsvariante besteht darin, z.B. die DNA eines beliebigen therapeutischen Gens und die cDNA von p21 auf verschiedenen Vektoren zu lokalisieren. Die Transfektion in die Zelle findet gleichzeitig statt.

In einer weiteren Variante kann der Vektor, der das Material zur Expression von p21 aufweist, zuerst in die Zielzelle eingebracht werden. Anschließend wird das Vektorsystem mit einem beliebigen Transgen übertragen, so daß die Expression von p21 bei Einführung des Vektors mit dem therapeutischen Material bereits stattfindet.

Die Erfindung betrifft weiterhin auch ein virales Vektorsystem zur Optimierung des Gentranfers, das gekennzeichnet ist
- durch die DNA eines viralen Vektors, der ein zu transferierendes genetisches Material und die cDNA für p21 aufweist oder
- durch die DNA eines viralen Vektors, der ein genetisches Material und die DNA eines weiteren Vektors, der die cDNA für p21 aufweist.

Besonders bevorzugt sind adenovirale Vektoren.

Die Erfindung wird am Beispiel der Verwendung eines adenoviralen Vektors näher erläutert.

Eines der häufigsten Vektorsysteme für den Gentransfer sind Adenoviren (Ad vektor). Sie sind besonders effizient und für in vivo- und in vitro-Gentranfer aufgrund hoher Titer und Stabilität im Blut besonders geeignet. Die natürlichen Zielzellen für Adenoviren sind die Zellen epitelialer Gewebe.

Nach Infektion von replizierenden oder nichtreplizierenden Zellen liegt das virale Genom im Zellkern epichromosomal vor, wodurch die Genexpression zeitlich begrenzt ist.

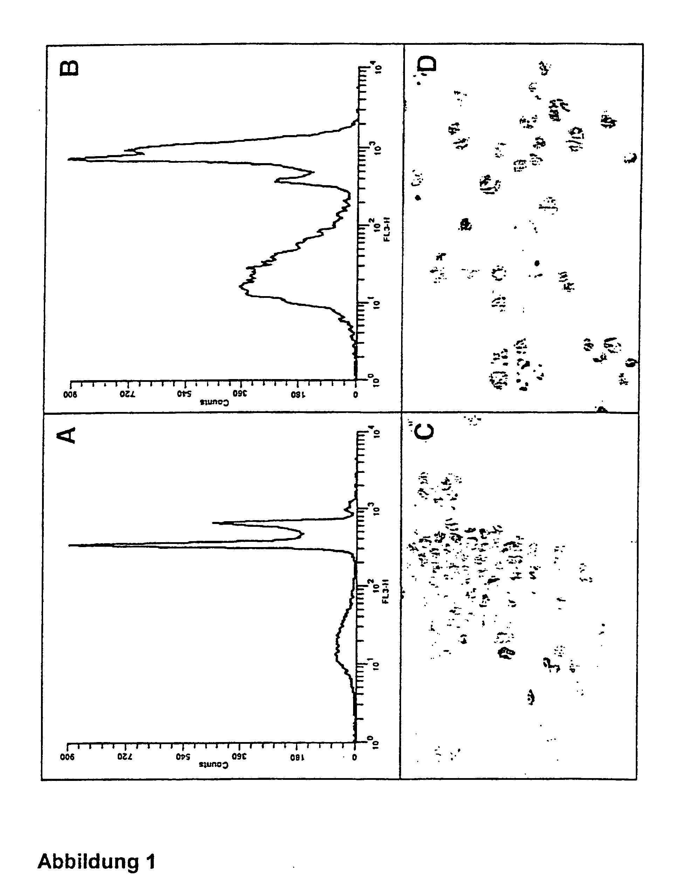

Abb. 1 zeigt am Beispiel eines Ad vektors, der das Protein alpha-1 Antitrypsin exprimiert, daß die Zellen durch Apoptose sterben. Diese erfolgt durch Aktivierung der S-Phase ohne folgende Mitose, was zu deformierten polyploiden Zellkernen führt.

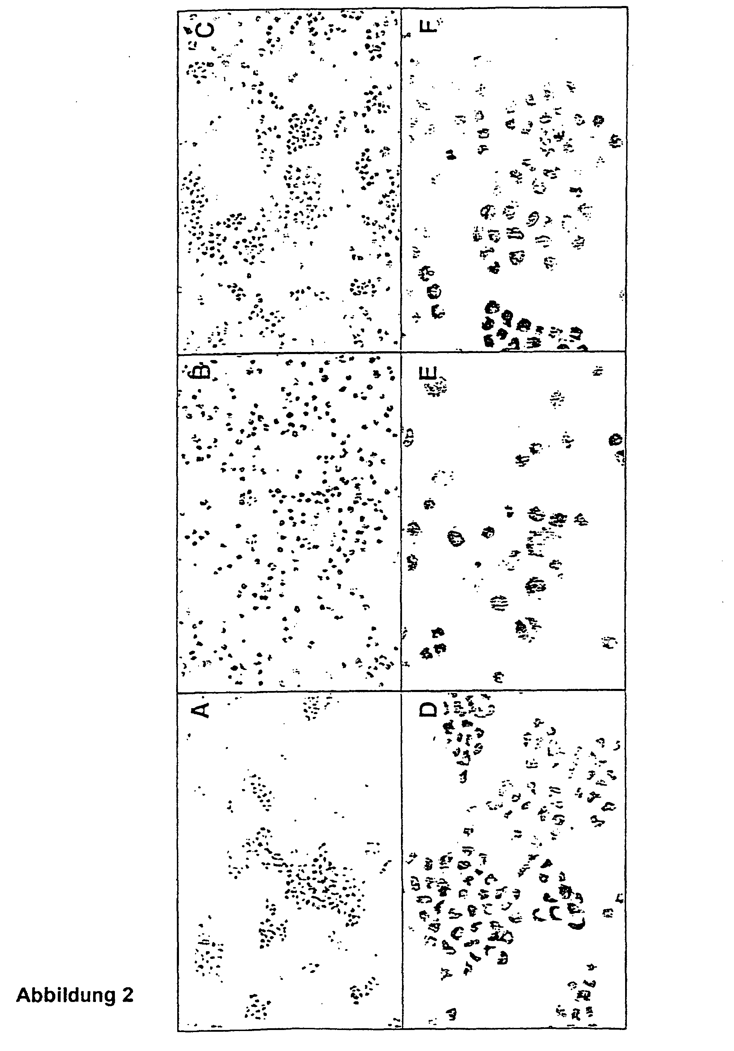

Erfindungsgemäß konnte nun die Apoptose der Zielzellen durch ektope Überproduktion, d.h. durch Koexpression des Zellzyklusregulators p21 verhindert werden. Unter Verwendung des gleichen Vektortyps waren die Zellen nach dem Gentransfer von p21 noch am Leben ohne Anzeichen deformierter polyploider Zellkerne und/oder Apoptose aufzuweisen (Abb. 2).

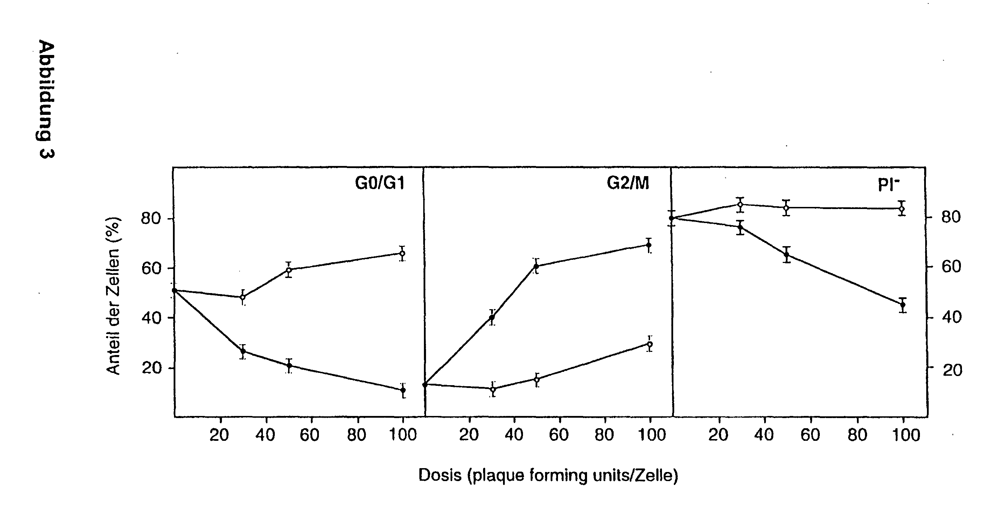

Der Abb. 3 kann entnommen werden, daß p21 tatsächlich die Zellen vor Apoptose bei Adenovirus-vermitteltem Gentransfer schützt. Dies wird anhand der Adenovirus-vermittelten Überexpression dargestellt, die die Aktivierung der S-Phase und Mitose verhindert.

### Legende zu den Abbildungen:

### Abb. 1

Replikationsdefiziente rekombinante Adenovirusvektoren induzieren Apoptose durch Entkoppeln von S-Phase und Mitose. Zellzyklusanalyse über Durchflußzytometrie (A, B) und in situ Apoptosenachweis über TUNEL-Assay (C, D) in LoVo Zellen 48 Stunden nach Virusinfektion. Zellen waren entweder pseudo-infiziert (Pufferkontrolle) (A, C) oder infiziert mit einem Ad vektor, der das alpha-1 Antitrypsingen trägt (100 plaque forming units/Zelle) (B, D).

### Abb. 2

Überexpression von p21 verhindert Adenovirus-induzierte Apoptose.
*In situ* Apoptosenachweis in LoVo Zellen 48 Stunden nach Infektion. Zellen waren entweder pseudo-infiziert (Pufferkontrolle) (A, D), infiziert mit einem Ad vektor (alpha-1 Antitrypsin; 100 plaque forming units/Zelle) (B, E) oder mit einem Ad vektor, der die cDNA für p21 trägt (100 plaque forming units/Zelle) (C, F). Vergrößerung 200fach (A-C) bzw. 600fach (D-F).

### Abb. 3

p21 schützt vor Adenovirus-induzierter Apoptose durch Verhinderung eines G2-ähnlichen Arrests.
Zellzyklusanalyse über Durchflußzytometrie von LoVo Zellen 48 Stunden nach Infektion mit verschiedenen Dosen von Ad vektoren, die entweder alpha-1 Antitrypsin (•) oder p21 (o) exprimieren. Dargestellt sind die relativen Anteile der Zellpopulationen, die sich in G0/G1 oder G2/M des Zellzyklus befinden, sowie der Prozentsatz der lebenden Zellen an der Gesamtzellzahl (PI⁻; nicht Propidiumiodid-gefärbt). Datenpunkte sind Mittelwerte ± Standardabweichung aus drei Experimenten.

## Patentansprüche

1. Verfahren zur Apoptosehemmung und damit zur Stabilisierung und Verbesserung des Gentransfers mittels viraler Vektoren von genetischem Material in Säugerzellen
**gekennzeichnet dadurch,**
**daß** das Gen oder dessen cDNA in der Zielzelle exprimiert wird unter gleichzeitiger Expression des Zellzyklusregulators p21.

2. Verfahren nach Anspruch 1,
**dadurch gekennzeichnet, daß**
der Gentransfer mittels an sich bekannter Gentechniken durch Übertragung der reinen DNA oder unter Verwendung viraler oder nichtviraler Vektoren erfolgt.

3. Verfahren nach Anspruch 1 oder 2,
**dadurch gekennzeichnet, daß**
der Gentransfer mittels retroviraler, adenoviraler, baculoviraler, parvoviraler, und/oder herpesviraler Vektoren erfolgt.

4. Verfahren nach Anspruch 1 bis 3,
**dadurch gekennzeichnet, daß**
das Transgen und die cDNA des p21 auf dem gleichen Vektor lokalisiert sind.

5. Verfahren nach Anspruch 1 bis 3,
**dadurch gekennzeichnet, daß**
das Transgen und die cDNA des p21 auf verschiedenen Vektoren lokalisiert sind.

6. Verfahren nach Anspruch 1 bis 5,
**dadurch gekennzeichnet, daß**
der Vektor, der das Material zur Expression von p21 aufweist zuerst in die Zielzelle eingebracht wird und anschließend das Vektorsystem mit dem Gen, so daß die Expression von p21 bei Einführung des Vektors mit dem genetischen Material bereits stattfindet.

7. Verwendung des Zellzyklusregulators p21 zur Herstellung von Arzneimitteln, die beim Gentransfer therapeutischer Gene mittels viraler Vektoren die Apoptose der Zielzelle verhindern.

## Claims

1. Method for apoptosis inhibition and thus for stabilisation and improvement of the gene transfer of genetic material by means of viral vectors in mammal cells
wherein
the gene or its cDNA is expressed in the target cell with simultaneous expression of the cell cycle regulator p21.

2. Method according to Claim 1,
wherein
the gene transfer is done by means of gene techniques known per se by transfer of the pure DNA or by making use of viral or non-viral vectors.

3. Method according to Claim 1 or 2,
wherein
the gene transfer is done by means of retroviral, adenoviral, baculoviral, parvoviral, and/or herpesviral vectors.

4. Method according to Claim 1 to 3,
wherein
the trans-gene and the cDNA of the p21 are localised on the same vector.

5. Method according to Claim 1 to 3,
wherein
the trans-gene and the cDNA of the p21 are localised on various vectors.

6. Method according to Claim 1 to 5,
wherein
the vector manifesting the material for expression of p21 is firstly inserted into the target cell and then the vector system with the gene, with the result that the expression of p21 takes place as early as the introduction of the vector with the genetic material.

7. Use of the cell cycle regulator p21 for the production of drugs which prevent the apoptosis of the target cells in the gene transfer of therapeutic genes by means of viral vectors.

## Revendications

1. Procédé destiné à inhiber l'apoptose et ainsi à stabiliser et améliorer le transfert des gènes d'un matériel génétique au moyen de vecteurs viraux dans des cellules de mammifères
**se caractérisant par le fait**
**que** le gène ou son cADN est exprimé dans la cellule cible en même temps que l'expression du régulateur du cycle cellulaire p21.

2. Procédé selon la revendication 1,
**se caractérisant par le fait que**
le transfert de gène se fait au moyen de techniques génétiques connues en soi par l'intermédiaire du transfert de l'ADN pur ou en employant des vecteurs viraux ou non viraux.

3. Procédé selon la revendication 1 ou 2,
**se caractérisant par le fait que**
le transfert de gène se fait au moyen des vecteurs de rétrovirus, d'adénovirus, de baculovirus, de parvovirus et/ou de virus de l'herpes.

4. Procédé selon la revendication 1 à 3,
**se caractérisant par le fait que**
le transgène et le cADN du p21 sont localisés sur le même vecteur.

5. Procédé selon la revendication 1 à 3,
**se caractérisant par le fait que**
le transgène et le cADN du p21 sont localisés sur différents vecteurs.

6. Procédé selon la revendication 1 à 5,
**se caractérisant par le fait que**
le vecteur qui dispose du matériel nécessaire pour exprimer p21, et d'abord intégré à la cellule cible et ensuite le système de vecteur avec le gène de sorte que l'expression de p21 ait déjà lieu lors de l'introduction du vecteur avec le matériel génétique.

7. Emploi du régulateur du cycle cellulaire p21 destiné à la fabrication de médicaments qui empêchent l'apoptose des cellules cibles lors du transfert génétique de gènes thérapeutiques au moyens de vecteurs viraux.
